# EUROPEAN PATENT APPLICATION

(11) **EP 4 404 202 A1**
(43) Date of publication of application: **24.07.2024**
(21) Application number: 24151197.1
(22) Date of filing: 10.01.2024
(51) Int. Cl.: G16B 40/20, G16H 50/20, G16H 30/40, G06N 3/045, G06T 7/00, G06V 10/82

(54) **METHOD AND DEVICE FOR GENERATING MEDICAL INFORMATION BASED ON PATHOLOGICAL SLIDE IMAGE**

(30) Priority: 19.01.2023 KR 20230008302; 26.05.2023 KR 20230068642
(71) Applicant: Lunit Inc., Seoul 06241 (KR)
(72) Inventor: PAENG, Kyung Hyun, 06241 Seoul (KR); OCK, Chan Young, 06241 Seoul (KR); YOO, Dong Geun, 06241 Seoul (KR)
(74) Representative: AWA Sweden AB

(57) **Abstract**

Provided is a computing device including at least one memory, and at least one processor configured to obtain feature information corresponding to a pathological slide image, generate medical information associated with the pathological slide image based on the feature information, and output at least one of the medical information and additional information based on the medical information.

## Description

### BACKGROUND

### 1. Field

The present disclosure relates to a method and device for generating medical information based on a pathological slide image.

### 2. Description of the Related Art

Digital pathology is a field for obtaining histological information of a patient or predicting a prognosis by using a whole slide image generated by scanning a pathological slide image.

The pathological slide image may be obtained from a stained tissue sample of an object. For example, a tissue sample may be stained by various staining methods, such as hematoxylin and eosin, trichrome, periodic acid-Schiff, autoradiography, enzyme histochemistry, immunofluorescence, and immunohistochemistry. The stained tissue sample may be used for histology and biopsy evaluations, and thus may operate as a basis for determining whether to move on to molecular profile analysis to understand a disease state.

Recently, technologies for predicting medical information about a subject by using medical images and machine learning models have been developed. In particular, technologies for analyzing cancer cells, cancer areas, and the like based on pathological slide images have been developed. In addition, as the relationship between gene mutations and cancer has been revealed, technologies for predicting the potential risk of cancer development in a subject by predicting gene mutations are emerging.

### SUMMARY

Provided are a method and device for generating medical information based on a pathological slide image. Provided is a computer-readable recording medium having recorded thereon a program for causing a computer to execute the method. The objectives of the present disclosure are not limited to those described above, and other objectives may be obtained.

Additional aspects will be set forth in part in the description which follows and, in part, will be apparent from the description, or may be learned by practice of the presented embodiments of the disclosure.

According to an aspect of an embodiment, a computing device includes at least one memory, and at least one processor configured to obtain feature information corresponding to a pathological slide image, generate medical information associated with the pathological slide image based on the feature information, and output at least one of the medical information and additional information based on the medical information.

According to an aspect of another embodiment, a method includes obtaining feature information corresponding to a pathological slide image, generating medical information associated with the pathological slide image based on the feature information, and outputting at least one of the medical information and additional information based on the medical information.

According to an aspect of another embodiment, a computer-readable recording medium includes a recording medium recording thereon a program for causing a computer to execute the above-described method.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a diagram for describing an example of a system for generating medical information from a pathological slide image, according to an embodiment;
FIG. 2A is a block diagram illustrating an example of a user terminal according to an embodiment;
FIG, 2B is a configuration diagram illustrating an example of a server according to an embodiment;
FIG. 3 is a flowchart for describing an example of a method of generating medical information from a pathological slide image, according to an embodiment;
FIG. 4 is a diagram for describing an example in which a processor obtains information about at least one object, according to an embodiment;
FIG. 5 is a diagram for example an example in which a processor obtains feature information, according to an embodiment;
FIG. 6 is a diagram for describing an example in which a processor generates medical information, according to an embodiment;
FIG. 7A is a diagram for describing an example in which an aggregator and a classifier operate, according to an embodiment;
FIG. 7B is a diagram for describing another example in which an aggregator and a classifier operate, according to an embodiment;
FIG. 7C is a diagram for describing another example in which an aggregator and a classifier operate, according to an embodiment;
FIG. 8 is a diagram for describing examples of medical information and additional information, according to an embodiment;
FIG. 9 is a diagram for describing an example in which a processor outputs medical information and/or additional information, according to an embodiment;
FIG. 10A is a diagram for describing an example in which a processor outputs a sequencing area, according to an embodiment;
FIG. 10B is a diagram for describing another example in which a processor outputs a sequencing area, according to an embodiment;
FIG. 10C is a diagram for describing another example in which a processor outputs a sequencing area, according to an embodiment;
FIG. 10D is a diagram for describing another example in which a processor outputs a sequencing area, according to an embodiment;
FIGS. 11A and 11B are diagrams illustrating examples of reports according to an embodiment;
FIG. 12 is a flowchart for describing another example of a method of generating medical information from a pathological slide image, according to an embodiment; and
FIG. 13 is a diagram for describing another example of a system for generating medical information from a pathological slide image.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

Terms used in embodiments are selected as currently widely used general terms as possible, which may vary depending on intentions or precedents of one of ordinary skill in the art, emergence of new technologies, and the like. In addition, in certain cases, there are also terms arbitrarily selected by the applicant, and in this case, the meaning thereof will be defined in detail in the description. Therefore, the terms used herein should be defined based on the meanings of the terms and the details throughout the present description, rather than the simple names of the terms.

Throughout the present specification, when a part "includes" a component, it means that the part may additionally include other components rather than excluding other components as long as there is no particular opposing recitation. In addition, as used herein, terms such as "... unit" or "... module" denote a unit that performs at least one function or operation, which may be implemented as hardware or software or a combination thereof.

In addition, although terms such as "first" or "second" may be used herein to describe various elements, these elements should not be limited by these terms. These terms may be only used to distinguish one element from another.

According to an embodiment, a "pathological slide image" may refer to an image obtained by photographing a pathological slide that is fixed and stained via a series of chemical treatment processes for tissue or the like removed from a human body. In addition, a pathological slide image may refer to a whole slide image (WSI) including a high-resolution image of a whole slide, and may also refer to a portion of the whole slide image, for example, one or more patches. For example, a pathological slide image may refer to a digital image captured or scanned via a scanning device (e.g., a digital scanner), and may include information about a particular protein, cell, tissue, and/or structure within a human body. In addition, the pathological slide image may include one or more patches, and histological information may be applied (e.g., tagged) to the one or more patches via an annotation operation.

"Medical information" may refer to any medically meaningful information that may be extracted from a medical image. For example, medical information may include at least one of an immune phenotype, a genotype, a biomarker score, tumor purity, information about ribonucleic acid (RNA), information about a tumor microenvironment, and a method of treating cancer expressed in a pathological slide image.

For example, the genotype may include a signature. The signature may refer to comprehensive information about one or more genes or their mutations expressed on a pathological slide image. For example, examples of genotypes may include genes or mutations such as Kirsten rat sarcoma viral (KRAS) or KRAS G12C. In addition, the genotype may be information associated with a mutation of an epidermal growth factor receptor (EGFR) gene, an anaplastic lymphoma kinase (ALK) fusion oncogene, a receptor tyrosine kinase (ROS1) oncogene, a kinesin family member 5B (KIF5B) gene, a receptor tyrosine kinase (RET) oncogene, a neurotrophic tyrosine kinase receptor (NTKR) oncogene, a breast cancer gene 1 (BRCA1) gene, a BRCA2 gene, an erb-B2 receptor tyrosine kinase 2 (ERBB2) gene, a B-Raf (BRAF) gene, MET, a MET proto oncogene, a serine/threonine kinase 11 (STK11) gene, a homologous recombination repair (HRR) pathway gene, mutations thereof, or treatment thereof.

In particular, a processor according to an embodiment may accurately predict a KRAS G12C mutation from a pathological slide image, and the KRAS G12C mutation may be used as a target for non-small cell lung cancer (NSCLC).

In addition, the genotypes in the present disclosure are not limited to the examples described above, and may include at least one of ABL1, ACVR1B, AKT1, AKT2, AKT3, ALK, ALOX12B, AMER1, APC, AR, ARAF, ARFRP1, ARID 1 A, ASXL1, ATM, ATR, ATRX, AURKA, AURKB, AXIN1, AXL, BAP1, BARD1, BCL2, BCL2L1, BCL2L2, BCL6, BCOR, BCORL1, BCR, BRAF, BRCA1, BRCA2, BRD4, BRIPI, BTG1, BTG2, BTK, C-Kit, Cllorf30, C17orf39, CAFR, CARD11, CASP8, CBFB, CBF, CCND1, CCND2, CCND3, CCNE1, CD22, CD70, CD74, CD79A, CD79B, CD274, CDC73, CDH1, CDK12, CDK4, CDK6, CDK8, CDKN1A, CDKN1B, CDKN2A, CDKN2B, CDKN2C, CEBPA, CHEK1, CHEK2, CIC, CREBBP, CRKL, CSF1R, CSF3R, CTCF, CTNNA1, CTNNB1, CUL3, CUL4A, CXCR4, CYP17A1, DAXX, DDR1, DDR2, DIS3, DNMT3A, DOT1L, EED, EGFR, EP300, EPHA3, EPHB1, EPHB4, ERBB2, ERBB3, ERBB4, ERCC4, ERG, ERRFI1, ESR1, ETV4, ETV5, ETV6, EWSR1, EZH2, EZR, FAM46C, FANCA, FANCC, FANCG, FANCL, FAS, FBXW7, FGF10, FGF12, FGF14, FGF19, FGF23, FGF3, FGF4, FGF6, FGFR1, FGFR2, FGFR3, FGFR4, FH, FFCN, FFT1, FFT3, FLT3, FOLR1, FOXF2, FUBP1, GABRA6, GAT A3, GATA4, GATA6, GNA11, GNA13, GNAQ, GNAS, GRM3, GSK3B, H3F3A, HDAC1, HER1, HER2, HGF, HLA, HNF1A, HRAS, HSD3B1, ID3, IDH1, IDH2, IGF1R, IKBKE, IKZF1, INPP4B, IRF2, IRF4, IRS2, JAK1, JAK2, JAK3, JUN, KDM5A, KDM5C, KDM6A, KDR, KEAP1, KEF, KIT, KI-67, KFHF6, KMT2A, KMT2D, KRAS, FTK, FYN, MAF,MAP2K1, MAP2K2, MAP2K4, MAP3K1, MAP3K13, MAPK1, MCF1, MDM2, MDM4, MED 12, MEF2B, MEN1, MERTK, MET, MITF, MKNK1, MEHI, MPE, MRE11A, MSH2, MSH3, MSH6, MST1R, MTAP, MTOR, MUTYH, MYB, MYC, MYCL, MYCN, MYD88, NBN, NF1, NF2, NFE2L2, NFKBIA, NKX2-1, NOTCH1, NOTCH2, NOTCH3, NPM1, NRAS, NSD3, NT5C2, NTRK1 NTRK2, NTRK3, NUTM1, P2RY8, PALB2, PARK2, PARP1, PARP2, PARP3, PAX5, PBRM1, PDCD1, PDCD1LG2, PDGFRA, PDGFRB, PDK1, PIK3C2B, PIK3C2G, PIK3CA, PIK3CB, PIK3R1, PIM1, PMS2, POLD1, POLE, PPARG, PPP2R1A, PPP2R2A, PRDM1, PRKAR1A, PRKCI, PTCH1, PTEN, PTPN11, PTPRO, QKI, RAC1, RAD21, RAD51, RAD51B, RAD51C, RAD51D, RAD52, RAD54L, RAF1, RARA, RBI, RBM10, REL, RET, RICTOR, RNF43, ROS1, RPTOR, RSP02, SDC4, SDHA, SDHB, SDHC, SDHD, SETD2, SF3B1, SGK1, SLC34A2, SMAD2, SMAD4, SMARCA4, SMARCB1, SMO, SNCAIP, SOCS1, SOX2, SOX9, SPEN, SPOP, SRC, STAG2, STAT3, STK11, SUFU, SYK, TBX3, TEK, TERC, TERT, TET2, TP53, TFGB, and/or TGFBR.

In addition, the medical information may include, but is not limited to, the area, location, and size of a particular tissue (e.g., a cancer tissue or a cancer stromal tissue) and/or a particular cell (e.g., a tumor cell, a lymphoid cell, a macrophage cell, an endothelial cell, or a fibroblast cell) in a medical image, cancer diagnosis information, information associated with a subject's possibility of developing cancer, and/or a medical conclusion associated with cancer treatment.

In addition, the medical information may include not only a quantified numerical value that may be obtained from a medical image, but also information obtained by visualizing the numerical value, predictive information according to the numerical value, image information, statistical information, and the like.

For example, the medical information may be provided to a user terminal or output through a display device.

Hereinafter, embodiments will be described in detail with reference to the accompanying drawings. The embodiments may, however, be embodied in many different forms and should not be construed as being limited to the embodiments set forth herein.

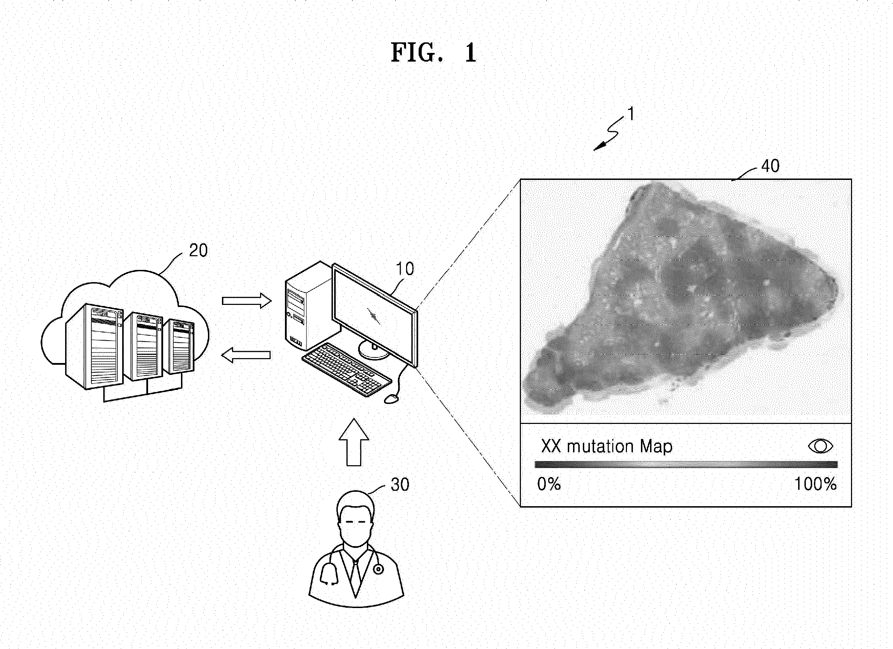

FIG. 1 is a diagram for describing an example of a system for generating medical information from a pathological slide image, according to an embodiment.

Referring to FIG. 1, a system 1 includes a user terminal 10 and a server 20. For example, the user terminal 10 and the server 20 may be connected to each other through by using a wired or wireless communication method, to transmit and receive various pieces of data to and from each other.

For convenience of description, FIG. 1 illustrates that the system 1 includes the user terminal 10 and the server 20, but the present disclosure is not limited thereto. For example, the system 1 may include other external devices (not shown). In addition, operations of the user terminal 10 and the server 20 to be described below may be implemented by a single device (e.g., the user terminal 10 or the server 20) or more devices.

The user terminal 10 may be a computing device that is provided with a display device and a device (e.g., a keyboard, a mouse, or the like) for receiving a user input, and includes a memory and a processor. In addition, the display device may be implemented as a touch screen to perform a function of receiving a user input. For example, the user terminal 10 may correspond to a notebook personal computer (PC), a desktop PC, a laptop, a tablet computer, a smart phone, or the like, but is not limited thereto.

The server 20 may be a device that communicates with an external device (not shown) including the user terminal 10. For example, the server 20 may be a device that stores various types of data including a pathological slide image, a bitmap image corresponding to a pathological slide image, information generated by analyzing a pathological slide image (including, for example, information about at least one object expressed in the pathological slide image, information about expression of at least one biomarker, information about a signature expressed by a combination of a plurality of biomarkers, medical information associated with a pathological slide image, additional information derived based on medical information, and the like), and information about a machine learning model used for analyzing a pathological slide image. Alternatively, the server 20 may be a computing device including a memory and a processor, and having a computing capability. In a case in which the server 20 is a computing device, the server 20 may perform at least some of operations of the user terminal 10 to be described below with reference to FIGS. 1 to 13. For example, the server 20 may be a cloud server, but is not limited thereto.

The user terminal 10 outputs an image 40 representing information generated by analyzing a pathological slide image and/or a pathological slide. For example, various pieces of information about at least one object expressed in the pathological slide image may be expressed in the image 40. In addition, biomarker expression information may be expressed in the image 40. In addition, medical information about at least a partial area included in the pathological slide image and/or additional information derived based on the medical information may be expressed in the image 40.

The pathological slide image may refer to an image obtained by photographing a pathological slide that is fixed and stained through a series of chemical treatment processes in order to observe, with a microscope, a tissue or the like removed from a human body. For example, the pathological slide image may refer to a whole slide image including a high-resolution image of a whole slide. As another example, the pathological slide image may refer to a part of such a high-resolution whole slide image.

In addition, the pathological slide image may refer to an area obtained by dividing the whole slide image into patches (or tiles). For example, the patch (or tile) may have a size of a certain region.

In addition, the pathological slide image may refer to a digital image captured by using a microscope, and may include information about cells, tissues, and/or structures in the human body.

Biological elements (e.g., cancer cells, immune cells, cancer areas, etc.) expressed in the pathological slide image may be identified by analyzing the pathological slide image. These biological elements may be used for histological diagnosis of a disease, prognosis of a disease, determination of a therapeutic direction for a disease, and the like.

Meanwhile, gene mutations or various biomarkers may serve as targets for particular cancer, or as important indicators in the diagnosis, treatment, and prognosis of cancer. For example, a KRAS G12C mutation may serve as a target for NSCLC, and accordingly, tissue-based KRAS mutation testing has been recognized as an essential test in deciding whether to treat NSCLC.

In general, pathologists determine medical information including gene mutations and various biomarkers based on pathological slide images, and draw clinical conclusions. However, a process of deriving medical information from a pathological slide image may be costly and time-consuming due to the possibility of subjective judgment by a pathologist on a clinical conclusion and the difficulty of ensuring the accuracy of judgment results.

The user terminal 10 according to an embodiment generates medical information associated with a pathological slide image, based on feature information corresponding to the pathological slide image. In addition, the user terminal 10 outputs at least one of the medical information and additional information based on the medical information.

Thus, the user terminal 10 may accurately and quickly generate medical information such as gene mutations or various biomarkers, and additional information based on the medical information, from a pathological slide image.

In addition, by combining information about an object extracted from the pathological slide image with feature information extracted from the pathological slide image, medical information may be generated by using various patterns and features in the pathological slide image. Thus, the medical information generated from the pathological slide image may be guaranteed to have high accuracy.

In addition, as the medical information includes information about various gene mutations (i.e., genotype information), targeted anti-cancer treatment may be performed quickly and accurately at a low cost. For example, the user terminal 10 may predict medical information (e.g., a KRAS G12C mutation) with high accuracy and stability by using a machine learning model and a pathological slide image. Thus, the medical information predicted by the user terminal 10 may be useful for next-generation sequencing (NGS) testing for targeted treatment of NSCLC or the like.

In addition, the user terminal 10 may generate a heat map or a distribution of various pieces of gene mutation information (i.e., genotype information), and generate a report including medical information and additional information. Thus, various pieces of information provided by the user terminal 10 may be useful for designing NGS testing or monitoring a patient in need of targeted anti-cancer treatment.

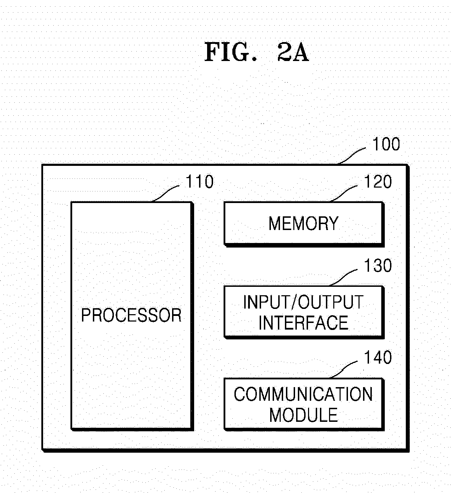

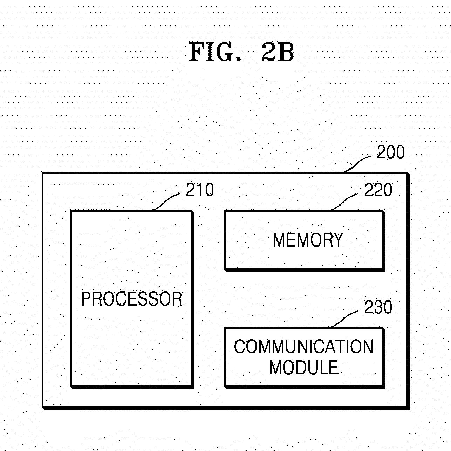

Hereinafter, an example in which the user terminal 10 generates medical information and/or additional information by using a pathological slide image will be described with reference to FIGS. 2 to 13.

Meanwhile, for convenience of description, it is described herein that the user terminal 10 performs all operations, but the present disclosure is not limited thereto. For example, at least some of operations performed by the user terminal 10 may also be performed by the server 20.

For example, the server 20 may obtain feature information corresponding to a pathological slide image. In addition, the server 20 may generate medical information associated with the pathological slide image based on the feature information. In addition, the server 20 may generate at least one of medical information and additional information based on the medical information, and transmit the generated information to the user terminal 10 and/or another external device (not shown). However, the operation of the server 20 is not limited to the above. FIG. 2A is a block diagram illustrating an example of a user terminal according to an embodiment.

Referring to FIG. 2A, a user terminal 100 includes a processor 110, a memory 120, an input/output interface 130, and a communication module 140. For convenience of description, FIG. 2A illustrates only components related to the present disclosure. Accordingly, other general-purpose components other than the components illustrated in FIG. 2A may be further included in the user terminal 100. In addition, it is obvious to those of skill in the art related to the present disclosure that the processor 110, the memory 120, the input/output interface 130, and the communication module 140 illustrated in FIG. 2A may also be implemented as independent devices.

The processor 110 may process commands of a computer program by performing basic arithmetic, logic, and input/output operations. Here, the commands may be provided from the memory 120 or an external device (e.g., the server 20). In addition, the processor 110 may control the overall operation of other components included in the user terminal 100.

The processor 110 obtains feature information corresponding to a pathological slide image. For example, the processor 110 may obtain first feature information based on information about at least one object expressed in the pathological slide image.

For example, the first feature information may include at least one feature vector that may be obtained by a feature embedding model. Here, the feature embedding model may be generated based on at least some layers of a first machine learning model configured to infer information about at least one object from a pathological slide image.

The processor 110 generates medical information associated with the pathological slide image, based on the first feature information. For example, the processor 110 may generate second feature information by combining the first feature information with information about an object, and generate medical information based on the second feature information. The second feature information may be another feature vector generated by combining at least one first feature vector with the information about the object.

Here, the processor 110 may perform pooling on at least one of the first feature vector, the information about the object, and a new feature vector that is the basis of the second feature vector.

In addition, the processor 110 may generate a new feature vector by combining the second feature vector and with a weight corresponding to the pathological slide image, and generate medical information based on the new feature vector.

The processor 110 outputs at least one of medical information and additional information based on the medical information. The processor 110 may generate additional information based on medical information, and output the medical information and/or the additional information.

For example, the processor 110 may control a display device to display, on a pathological slide image, information indicating at least one genomic variation expressed in the pathological slide image.

As another example, the processor 110 may control the display device to display, on a pathological slide image, a sequencing area that is set based on medical information. Here, the sequencing area may be set adaptively for each method of separating a sequencing area from a subject. For example, the processor 110 may set the sequencing area differently depending on a method of separating and dissecting a part of the subject on which sequencing is to be performed. In addition, the processor 110 may control the display device to further output an expected yield of mutation when separating the sequencing area from the subject.

As another example, the processor 110 may generate a report including the sequencing area that is set based on the medical information, and detailed information about the sequencing area.

As another example, the processor 110 may determine a probability of detecting a particular gene mutation from a subject based on medical information, and based on the probability being greater than or equal to a threshold value, may transmit information about the sequencing area to another external device.

The processor 110 may be implemented as an array of a plurality of logic gates, or may be implemented as a combination of a general-purpose microprocessor and a memory storing a program executable by the microprocessor. For example, the processor 110 may include a general-purpose processor, a central processing unit (CPU), a microprocessor, a digital signal processor (DSP), a controller, a microcontroller, a state machine, and the like. In some environments, the processor 110 may include an application-specific integrated circuit (ASIC), a programmable logic device (PLD), a field-programmable gate array (FPGA), and the like. For example, processor 110 may refer to a combination of processing devices, such as a combination of a DSP and a microprocessor, a combination of a plurality of microprocessors, a combination of one or more microprocessors combined with a DSP core, or a combination of any other such configurations.

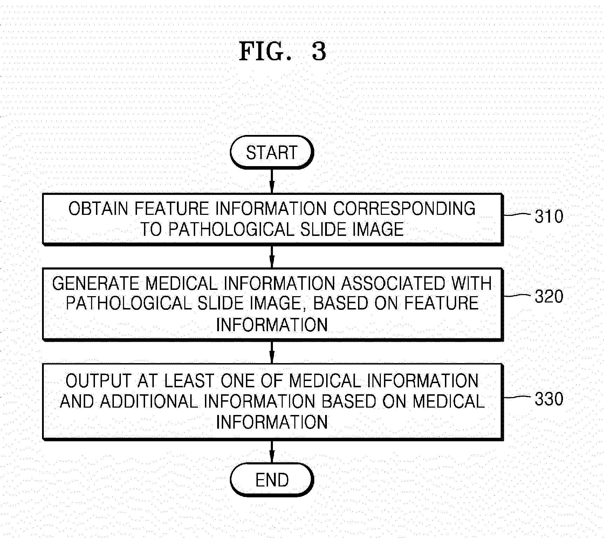

The memory 120 may include any non-transitory computer-readable recording medium. For example, the memory 120 may include a permanent mass storage device, such as random-access memory (RAM), read-only memory (ROM), a disk drive, a solid-state drive (SSD), or flash memory. As another example, the permanent mass storage device, such as ROM, an SSD, flash memory, or a disk drive, may be a permanent storage device separate from the memory. Also, the memory 120 may store an operating system (OS) and at least one piece of program code (e.g., code for the processor 110 to perform an operation to be described below with reference to FIGS. 3 to 13).

These software components may be loaded from a computer-readable recording medium separate from the memory 120. The separate computer-readable recording medium may be a recording medium that may be directly connected to the user terminal 100, and may include, for example, a computer-readable recording medium, such as a floppy drive, a disk, a tape, a digital video disc (DVD)/compact disc ROM (CD-ROM) drive, or a memory card. Alternatively, the software components may be loaded into the memory 120 through the communication module 140 rather than a computer-readable recording medium. For example, at least one program may be loaded to the memory 120 based on a computer program (e.g., a computer program for the processor 110 to perform an operation to be described below with reference to FIGS. 3 to 13) installed by files provided by developers or a file distribution system that provides an installation file of an application, through the communication module 140.

The input/output interface 130 may be a unit for an interface with a device (e.g., a keyboard or a mouse) for input or output that may be connected to the user terminal 100 or included in the user terminal 100. Although FIG. 2A illustrates that the input/output interface 130 is a component implemented separately from the processor 110, the present disclosure is not limited thereto, and the input/output interface 130 may be implemented to be included in the processor 110.

The communication module 140 may provide a configuration or function for a server 200 and the user terminal 100 to communicate with each other through a network. In addition, the communication module 140 may provide a configuration or function for the user terminal 100 to communicate with another external device. For example, a control signal, a command, data, and the like provided under control of the processor 110 may be transmitted to the server 20 and/or an external device through the communication module 140 and a network.

Meanwhile, although not illustrated in FIG. 2A, the user terminal 100 may further include a display device. Alternatively, the user terminal 100 may be connected to an independent display device by a wired or wireless communication method to transmit and receive data to and from the display device. For example, a pathological slide image, information obtained by analyzing the pathological slide image, medical information, additional information based on the medical information, and the like may be provided to a user 30 through the display device.

FIG, 2B is a configuration diagram illustrating an example of a server according to an embodiment.

Referring to FIG. 2B, the server 200 includes a processor 210, a memory 220, and a communication module 230. For convenience of description, FIG. 2B illustrates only components related to the present disclosure. Thus, other general-purpose components other than those illustrated in FIG. 2B may be further included in the server 200. In addition, it is obvious to those of skill in the art related to the present disclosure that the processor 210, the memory 220, and the communication module 230 illustrated in FIG. 2B may also be implemented as independent devices.

The processor 210 may obtain a pathological slide image from at least one of the internal memory 220, the user terminal 100, and another external device. The processor 210 may analyze at least one object expressed in the pathological slide image, obtain feature information corresponding to the pathological slide image, generate medical information associated with the pathological slide image based on the feature information, or transmit at least one of the medical information and additional information to the user terminal 100.

In other words, at least one of the operations of the processor 110 described above with reference to FIG. 2A may be performed by the processor 210. In this case, the user terminal 100 may output, through the display device, information transmitted from the server 200.

Meanwhile, an implementation example of the processor 210 is the same as that of the processor 110 described above with reference to FIG. 2A, and thus, detailed descriptions thereof will be omitted.

The memory 220 may store various pieces of data, such as a pathological slide image or data generated according to an operation of the processor 210. In addition, the memory 220 may store an OS and at least one program (e.g., a program required for the processor 210 to operate).

Meanwhile, an implementation example of the memory 220 is the same as that of the memory 120 described above with reference to FIG. 2A, and thus, detailed descriptions thereof will be omitted.

The communication module 230 may provide a configuration or function for the server 200 and the user terminal 100 to communicate with each other through a network. In addition, the communication module 230 may provide a configuration or function for the server 200 to communicate with another external device. For example, a control signal, a command, data, and the like provided under control of the processor 210 may be transmitted to the user terminal 100 and/or an external device through the communication module 230 and a network.

FIG. 3 is a flowchart for describing an example of a method of generating medical information from a pathological slide image, according to an embodiment.

The method illustrated in FIG. 3 includes operations processed in a time-series manner by the user terminal 10 or 100 or the processor 110 illustrated in FIGS. 1 and 2A. Thus, the descriptions provided above with respect to the user terminal 10 or 100 or the processor 110 illustrated in FIGS. 1 and 2A, which are even omitted below, may also be applied to the method illustrated in FIG. 3.

In addition, as described above with reference to FIGS. 1 to 2B, at least one of operations of the method illustrated in FIG. 3 may be processed by the server 20 or 200 or the processor 210.

In operation 310, the processor 110 obtains feature information corresponding to a pathological slide image.

In the present disclosure, a pathological slide image may be a whole slide image or a portion of a whole slide image. Here, some pathological slide images may be referred to as patches or tiles, which refer to partial images of whole slide images. For example, a patch or a tile may be an image representing a certain object, or may be an image representing a region of interest designated by the user 30.

For example, the processor 110 may obtain information about at least one object by analyzing the pathological slide image. In addition, the processor 110 may obtain feature information based on the information about the at least one object.

Here, the object may include cells, tissues, structures, and the like that constitute a subject. For example, the processor 110 may classify and extract a cancer area, a cancer stroma area, a necrosis area, a background area, and the like, from the pathological slide image. In addition, the processor 110 may classify a plurality of cells expressed in the pathological slide image into at least one of tumor cells, lymphocyte cells, and other cells.

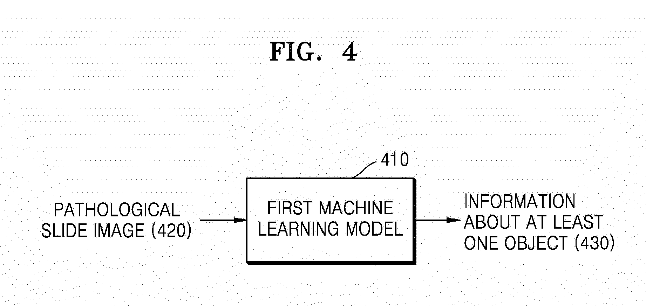

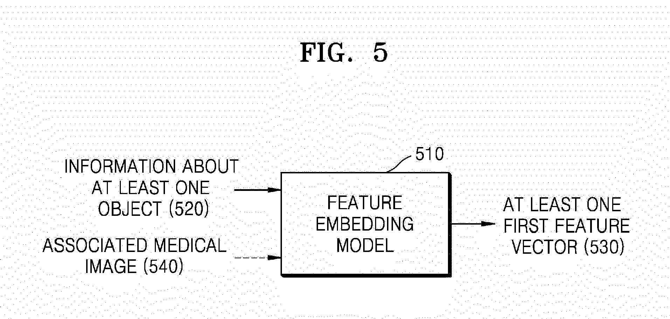

Meanwhile, the 'feature information' may correspond to information 430 about at least one object to be described below with reference to FIG. 4, and may correspond to at least one first feature vector 530. Hereinafter, an example in which the processor 110 obtains feature information will be described with reference to FIGS. 4 and 5.

FIG. 4 is a diagram for describing an example in which a processor obtains information about at least one object, according to an embodiment.

Referring to FIG. 4, the processor 110 may obtain the information 430 about at least one object by using a first machine learning model 410. For example, the first machine learning model 410 may be trained to output the information 430 about at least one object in response to receiving an input of a pathological slide image 420.

Any image from which the information 430 about at least one object may be extracted may correspond to the pathological slide image 420 without limitation. For example, the pathological slide image 420 may refer to an image stained by using one or more methods (e.g., a haematoxylin-and-eosin (H&E)- stained image or an immunohistochemistry (IHC)-stained image). Meanwhile, in the present specification, it is assumed that pathological slide images are subjects to be analyzed, but the present disclosure is not limited thereto. For example, the processor 110 according to an embodiment may analyze not only pathological slide images but also various types of medical images of a subject's body (e.g., a chest X-ray (CXR), a mammogram (MMG), an unstained slide image, or a radiograph) instead of the pathological slide image 420.

The information 430 about at least one object may include information about tissues and/or information about cells. However, the information 430 about at least one object is not limited thereto, and may include any visual information contained in the pathological slide image 420 without limitation. For example, the information 430 about at least one object may include various pieces of morphological information expressed in the pathological slide image 420.

For example, the processor 110 may analyze the pathological slide image 420 to classify a plurality of tissues expressed in the pathological slide image 420, by using the first machine learning model 410.

First, the processor 110 may detect areas corresponding to tissues, from the pathological slide image 420. The processor 110 may output a result of the detecting in the form of layers representing tissues, by using the machine learning model. In this case, the machine learning model may be trained, by using training data including a plurality of reference pathological slide images and a plurality of pieces of reference label information, to detect areas corresponding to tissues in the reference pathological slide images.

In addition, the processor 110 may classify the plurality of tissues expressed in the pathological slide image 420 by using the first machine learning model 410. For example, the processor 110 may classify the pathological slide image 420 into at least one of a cancer area, a cancer stroma area, a necrosis area, and a background area. Here, the background area may include an area representing biological noise and/or an area representing technical noise. For example, the area representing the biological noise may include a normal area, and the area representing the technical noise may include a degradation area.

However, examples in which the processor 110 classifies at least a partial area expressed in the pathological slide image 420 are not limited to the above description. In other words, without being limited to the above-described four types of areas (i.e., the cancer area, the cancer stroma area, the necrosis area, and the background area), the processor 110 may classify at least one area expressed in the pathological slide image 420 into a plurality of categories according to various criteria. At least one area expressed in the pathological slide image 420 may be classified into a plurality of categories according to a preset criterion or a criterion set by a user. In addition, the type of noise is not limited to the biological noise and the technical noise.

As another example, the processor 110 may analyze the pathological slide image 420 to classify a plurality of cells expressed in the pathological slide image 420, by using the first machine learning model 410.

First, the processor 110 may detect cells from the pathological slide image 420 by using the first machine learning model 410, and output a result of the detecting in the form of layers representing the cells. A detailed method of analyzing the pathological slide image 420 by the processor 110 is the same as described above.

In addition, the processor 110 may classify the plurality of cells expressed in the pathological slide image 420 by using the first machine learning model 410. For example, the processor 110 may classify cells expressed in the pathological slide image 420 into at least one of tumor cells, lymphocyte cells, and other cells. However, an example in which the processor 110 classifies the cells expressed in the pathological slide image 420 is not limited to the above description. In other words, the processor 110 may group the cells expressed in the pathological slide image 420 according to various criteria for classifying different types of cells.

In the present disclosure, a machine learning model refers to a statistical learning algorithm implemented based on the structure of a biological neural network, or a structure for executing the algorithm. For example, the machine learning model may refer to a machine learning model that obtains a problem-solving ability by repeatedly adjusting the weights of synapses by nodes that are artificial neurons forming a network in combination with the synapses as in biological neural network, to be trained such that an error between a correct output corresponding to a particular input and an inferred output is reduced. For example, the machine learning model may include an arbitrary probability model, a neural network model, and the like used in artificial intelligence learning methods, such as machine learning or deep learning.

For example, the machine learning model may be implemented as a multilayer perceptron (MLP) composed of multilayer nodes and connections therebetween. The machine learning model according to the present embodiment may be implemented by using one of various artificial neural network model structures including MLP. For example, the machine learning model may include an input layer that receives an input signal or data from the outside, an output layer that outputs an output signal or data corresponding to the input data, and at least one hidden layer that is between the input layer and the output layer, and receives a signal from the input layer, extracts features, and delivers the features to the output layer. The output layer receives a signal or data from the hidden layer, and outputs the signal or data to the outside.

Thus, the machine learning model may be trained to receive one or more pathological slide images and extract information about at least one object (e.g., cells, tissues, or structures) included in the pathological slide images.

As described above, the processor 110 according to an embodiment may generate the information 430 about at least one object, from the pathological slide image 420. The processor 110 according to an embodiment may finally generate medical information and additional information based on the medical information, by using the information 430 about at least one object.

In addition, the processor 110 according to an embodiment may generate the medical information and the additional information based on the medical information, by using not only the information 430 about at least one object but also at least one feature vector extracted from the pathological slide image. This will be described in detail below.

FIG. 5 is a diagram for example an example in which a processor obtains feature information, according to an embodiment.

Referring to FIG. 5, the processor 110 may obtain at least one first feature vector 530 by using a feature embedding model 510. For example, the feature embedding model 510 may generate the at least one first feature vector 530 by using information 520 about at least one object.

The feature embedding model 510 refers to a model that generates a feature vector in which the information 520 about at least one object is abstracted with semantic information. Here, the information 520 about at least one object corresponds to the information 430 illustrated in FIG. 4, and includes morphological information expressed in the pathological slide image 420.

For example, the feature embedding model 510 may be generated based on at least some layers of the first machine learning model 410. As described above with reference to FIG. 4, the first machine learning model 410 is a model that infers the information 430 about at least one object from the pathological slide image 420. The feature embedding model 510 may be implemented as a model obtained by removing one or more layers from among a plurality of layers constituting the first machine learning model 410.

As another example, the feature embedding model 510 may be a separate machine learning model from the first machine learning model 410. For example, the feature embedding model 510 may be implemented by removing one or more layers from a neural network model trained to extract morphological information from an arbitrary pathological slide image.

Meanwhile, for generating the feature information 530, an associated medical image 540 may be further used. In other words, the feature embedding model 510 may generate the feature information 530 by using the information 520 about at least one object and the associated medical image 540.

For example, the associated medical image 540 may be the same as the pathological slide image 420.

As another example, the associated medical image 540 may be different from the pathological slide image 420. For example, the associated medical image 540 may express the same object as in the pathological slide image 420, but may be an image stained by using a different staining method from that used for the pathological slide image 420, or may be of a different type from that of the pathological slide image 420.

Referring again to FIG. 3, in operation 320, the processor 110 generates medical information associated with the pathological slide image, based on the feature information.

The processor 110 may generate a second feature vector by combining the first feature vector included in the feature information, with information about at least one object expressed in the pathological slide image. In addition, the processor 110 may generate medical information based on the second feature vector.

For example, the processor 110 may generate the second feature vector by combining the first feature vector with the information about the object through an aggregator. Here, the first feature vector and the information about the object that are combined by the aggregator may be data corresponding to the same position on the pathological slide image.

In addition, the processor 110 may generate medical information from the second feature vector through a classifier. For example, the classifier may be trained to extract medical information from a second feature vector.

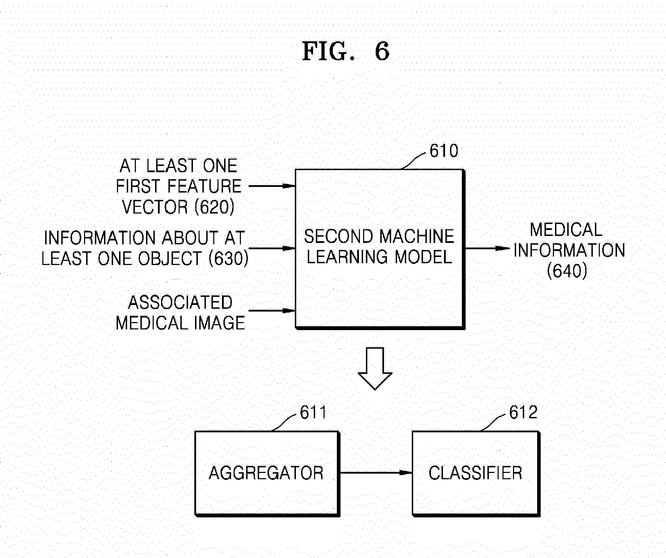

Hereinafter, an example in which the processor 110 generates medical information will be described with reference to FIGS. 6 to 7C.

FIG. 6 is a diagram for describing an example in which a processor generates medical information, according to an embodiment.

Referring to FIG. 6, the processor 110 may generate medical information 640 through a second machine learning model 610. The second machine learning model 610 may be trained to predict the medical information 640 in response to receiving an input of feature information 620 and information 630 about at least one object. In addition, an associated medical image may be further input to the second machine learning model 610 to predict the medical information 640. Here, the feature information 620 and the information 630 about at least one object correspond to the feature information 530 illustrated in FIG. 5 and the information 430 about at least one object illustrated in FIG. 4, respectively.

Meanwhile, the second machine learning model 610 may include an aggregator 611 and a classifier 612.

The aggregator 611 performs an operation of integrating pieces of information having various sizes into one vector having a fixed size. Medical images, including pathological slide images, may have various sizes, and the number of patches (tiles) or the number of first feature vectors may also vary. Accordingly, as the aggregator 611 integrates the first feature vector 620 of various specifications and the information 630 about at least one object into a second feature vector having a preset size, the processor 110 may be universally used for various medical images.

The classifier 612 serves to generate medical information from the second feature vector. For example, the classifier 612 may be implemented by an algorithm such as MLP, support vector machine (SVM), random forest, or decision tree. However, examples of the classifier 612 are not limited thereto, and the classifier 612 may be implemented as any unit as long as it is capable of extracting medical information by using a second feature vector.

Meanwhile, the processor 110 may perform pooling on at least one of the at least one first feature vector 620, the information 630 about at least one object, and a new feature vector. Here, the new feature vector refers to a vector that is the basis of the second feature vector. In addition, at least one of the aggregator 611 and the classifier 612 may operate based on learning.

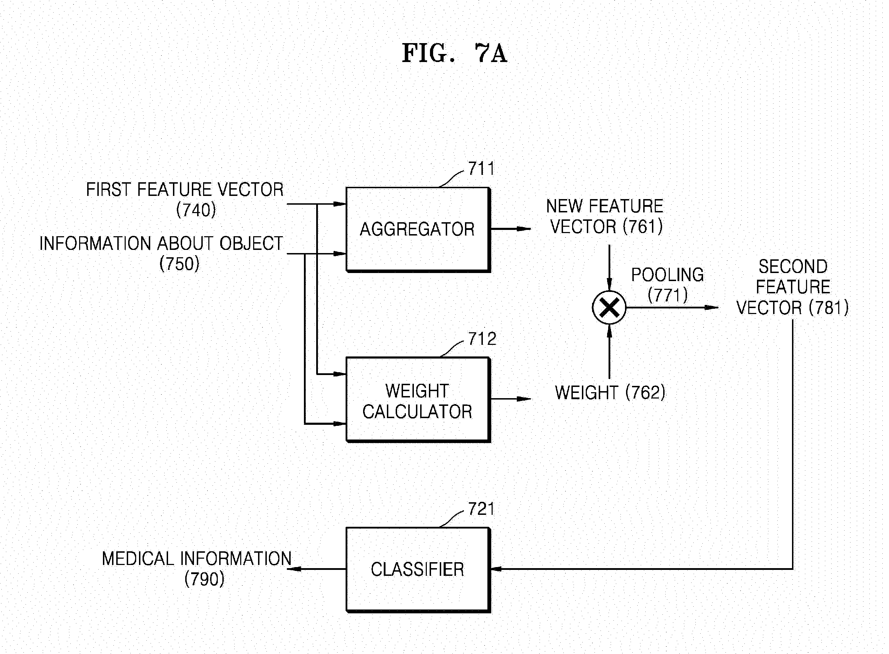

Hereinafter, examples in which the aggregator 611 and the classifier 612 operate will be described with reference to FIGS. 7A to 7C. In FIGS. 7A to 7C, it is assumed that a pathological slide image is divided into a plurality of tiles (patches), and a first feature vector and information about an object are obtained from each tile (patch).

FIG. 7A is a diagram for describing an example in which an aggregator and a classifier operate, according to an embodiment.

FIG. 7A illustrates an example in which an aggregator 711 generates a new feature vector 761 by combining a first feature vector 740 with information 750 about an object, a second feature vector 781 is generated by performing pooling 771 on a result of combining the new feature vector 761 with a weight 762, and a classifier 721 generates medical information 790 based on the second feature vector 781. For example, each of the aggregator 711 and the classifier 721 may be implemented as a deep neural network (DNN), and may operate based on learning.

The aggregator 711 generates the new feature vector 761 by combining the first feature vector 740 with the information 750 about the object. Here, the first feature vector 740 and the information 750 about the object are obtained for each of a plurality of tiles (patches) constituting the pathological slide image. Thus, in a case in which the pathological slide image is divided into n tiles (patches), n new feature vectors 761 are generated.

A weight calculator 712 calculates the weight 762 based on the first feature vector 740 and the information 750 about the object. Here, the weight 762 correspond to the pathological slide image. In detail, the weight 762 may be generated to correspond to each of a plurality of tiles (patches) included in the pathological slide image. Because the first feature vector 740 and the information 750 about the object exist for each of the plurality of tiles (patches), the weight 762 is also generated for each tile (patch). Here, the weight 762 may be determined based on the importance of the corresponding tile.

The processor 110 generate the second feature vector 781 by combining the new feature vector 761 with the weight 762 and performing the pooling 771. For example, the processor 110 may generate the second feature vector 781 by multiplying, for each tile (patch), the new feature vector 761 by the weight 762, performing the pooling 771 on each of results of the multiplying, and summing up results of the pooling 771. Accordingly, the second feature vector 781 having a fixed size may be generated from the first feature vector 740 having an undetermined size and the information 750 about the object.

For example, the pooling 771 may correspond to at least one of average pooling, max pooling, variance pooling, and bag-of-words (BOW)/Fisher kernel/vector of locally aggregated descriptors (VLAD) pooling, but is not limited thereto.

The classifier 721 predicts the medical information 790 based on the second feature vector 781.

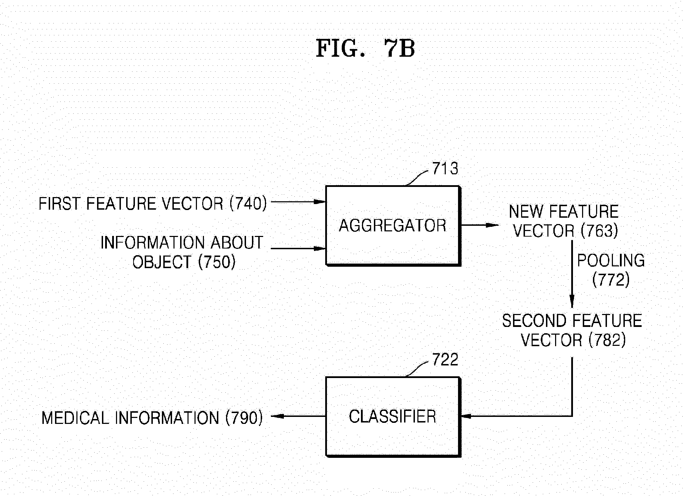

FIG. 7B is a diagram for describing another example in which an aggregator and a classifier operate, according to an embodiment.

FIG. 7B illustrates an example in which an aggregator 713 generates a new feature vector 763 by combining the first feature vector 740 with the information 750 about the object, a second feature vector 782 is generated by performing pooling 772 on the new feature vector 763, and a classifier 722 generates the medical information 790 based on the second feature vector 782. For example, the classifier 722 may be implemented as a DNN, and may operate based on learning.

The aggregator 713 generates the new feature vector 763 by combining the first feature vector 740 with the information 750 about the object. Here, an example of generating the new feature vector 763 is the same as described above with reference to FIG. 7A.

The processor 110 generates the second feature vector 782 by performing the pooling 772 on the new feature vector 763. For example, the processor 110 may generate the second feature vector 782 by performing the pooling 772 on the new feature vector 761 generated for each tile (patch), and summing up results of the pooling 772. Accordingly, the second feature vector 782 having a fixed size may be generated from the first feature vector 740 having an undetermined size and the information 750 about the object. Here, an example of the pooling 772 is as described above with reference to FIG. 7A.

The classifier 721 predicts the medical information 790 based on the second feature vector 782.

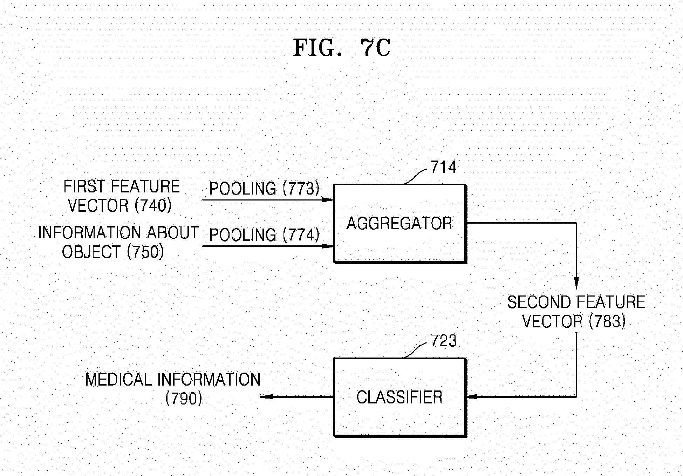

FIG. 7C is a diagram for describing another example in which an aggregator and a classifier operate, according to an embodiment.

FIG. 7C illustrates an example in which pooling 773 and 774 is performed on the first feature vector 740 and the information 750 about the object, respectively, an aggregator 714 generates a second feature vector 783 by combining results of the pooling, and a classifier 723 generates the medical information 790 based on the second feature vector 783. For example, the classifier 723 may be implemented as a DNN, and may operate based on learning. In addition, examples of the pooling 773 and 774 are as described above with reference to FIG. 7A.

The aggregator 713 generates the second feature vector 783 by combining the first feature vector on which the pooling has been performed, with the information about the object on which the pooling has been performed. Accordingly, the second feature vector 783 having a fixed size may be generated from the first feature vector 740 having an undetermined size and the information 750 about the object.

The classifier 723 predicts the medical information 790 based on the second feature vector 783.

Meanwhile, although not illustrated in FIGS. 7A to 7C, the processor 110 may infer a tile (patch) that has the greatest influence on predicting the medical information 790, from among the tiles (patches). In addition, the processor 110 may extract information for configuring a heat map to be described below, from the tile (patch) determined through the inference.

Referring again to FIG. 3, in operation 330, the processor 110 outputs at least one of the medical information and additional information based on the medical information.

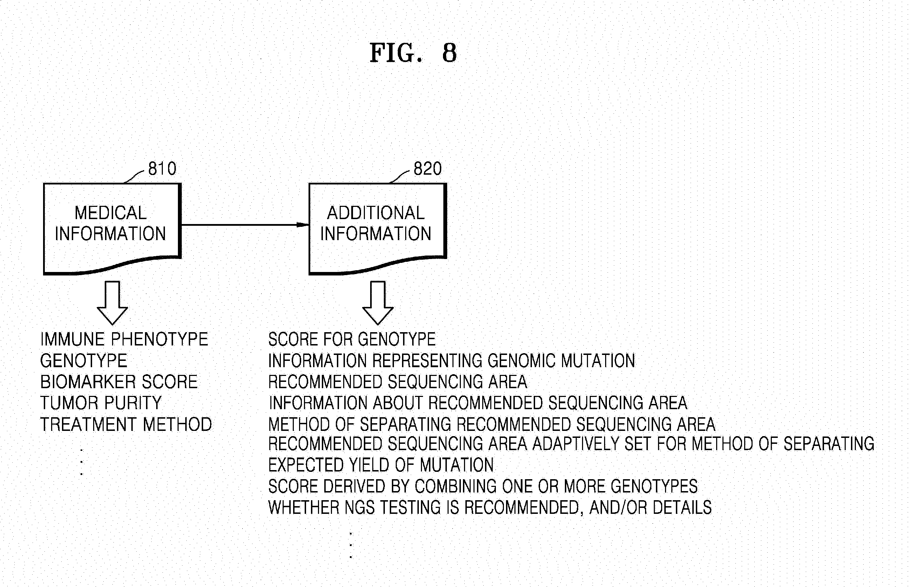

For example, the medical information may include at least one of an immune phenotype, a genotype, a biomarker score, tumor purity, and a method of treating cancer expressed in the pathological slide image. In addition, the additional information may include various pieces of information that may be derived based on the medical information. Hereinafter, examples of additional information will be described with reference to FIG. 8.

FIG. 8 is a diagram for describing examples of medical information and additional information, according to an embodiment.

FIG. 8 illustrates examples of medical information 810 and additional information 820. However, the medical information 810 and the additional information 820 are not limited to the examples illustrated in FIG. 8. In addition to the examples illustrated in FIG. 8, the medical information 810 may include various pieces of information that may be inferred based on a feature vector (i.e., a second feature vector) corresponding to a pathological slide image. In addition, in addition to the examples illustrated in FIG. 8, various pieces of information that may be derived from the medical information 810 may be included in the additional information 820.

For example, the additional information 820 may include a genotype score, information representing a genomic mutation (e.g., an expression rate or expression state information), a recommended sequencing area, information about the recommended sequencing area (e.g., yield or shape), a method of separating the recommended sequencing area (e.g., scalpel, punch, or microdissection), a recommended sequencing area set differently (i.e. adaptively) for the method of separating, an expected yield of mutation, a score derived by combining one or more genotypes, whether NGS testing is recommended, and/or details (e.g., 'NGS highly recommended', 'NGS recommended', or 'Need continuous observation'). In addition, the additional information 820 may include other quantitative information (e.g., a HER2 score or a PD-L1 score), other bibliographic information (e.g., personal information, examination time, or cancer type), the pathological slide image, a heat map for a particular genotype, and the like.

The processor 110 may output the medical information 810 and/or the additional information 820 in various ways. In detail, the processor 110 may control the display device to output the medical information 810 and/or the additional information 820. For example, the processor 110 may output the information 810 and 820 as text or an image (e.g., a heat map). In addition, the output as text may include output in the form of a report.

Meanwhile, the medical information 810 and the additional information 820 are separately illustrated in FIG. 8, but the present disclosure is not limited thereto. In other words, the medical information 810 and the additional information 820 may be collectively referred to as the medical information 810, and all examples included in the additional information 820 may also be included in the medical information 810. Thus, hereinafter, both the medical information 810 and the additional information 820 will be collectively referred to as the medical information 810.

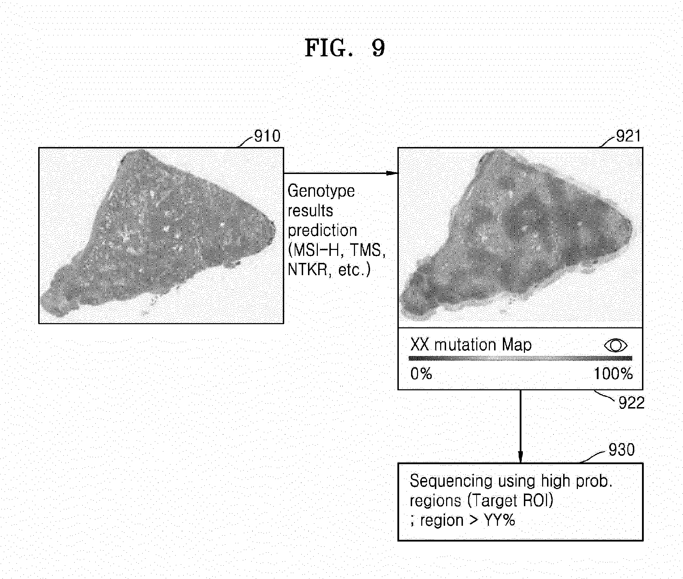

Hereinafter, examples in which the processor 110 outputs the medical information 810 and/or the additional information 820 will be described with reference to FIGS. 9 to 11B.

FIG. 9 is a diagram for describing an example in which a processor outputs medical information and/or additional information, according to an embodiment.

FIG. 9 illustrates a pathological slide image 910 expressing at least one subject, and various pieces of medical information 921, 922, and 930. For example, genotype information may include scores for several genotypes for the pathological slide image 910.

The genotype score may be a probability value that a mutation of the corresponding gene will be detected when a genomic test is actually performed on a subject. For example, a genotype A score may be expressed as 60, and a genotype B score may be expressed as 30. Here, A and B refer to values for single gene mutations such as KRAS or MSI.

In addition, the genotype scores may include result values for various genotypes, and may also include scores for complex values (e.g., TMB or HRD) rather than single gene mutations.

The processor 110 may output information indicating at least one genomic variation expressed in the pathological slide image 910. For example, the genomic variation may be calculated into a genotype score, and the processor 110 may output the genotype score in various ways.

For example, the processor 110 may output genotype scores as a heat map 921. The processor 110 may generate the heat map 921 by mapping the genotype scores to respective colors (922), and displaying the colors on the pathological slide image 910. Thus, the user 30 may view the heat map 921 to recognize the genotype scores and perform spatial analysis of the genomic variation.

In addition, the processor 110 may also output information 930 about what percentage of the total area of an object expressed in the pathological slide image 910 an area to be sequenced occupies.

Meanwhile, although not illustrated in FIG. 9, the processor 110 may output a map representing tumor purity for each area of the pathological slide image 910. In addition, the processor 110 may provide a viewer through which the heat map 921 and/or the map representing the tumor purity may be observed from various points of view through manipulation by the user 30.

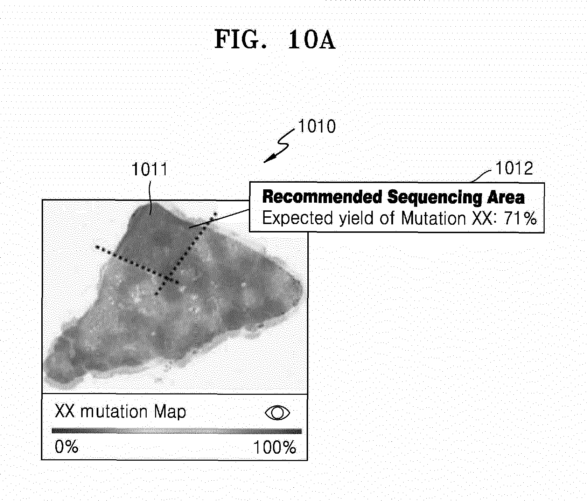

Meanwhile, the processor 110 may set a sequencing area based on the medical information (e.g., a genotype score), and display the set sequencing area on the pathological slide image. Because the heat map 921 is expressed in colors corresponding to genotype scores, an area to be sequenced may be set. The processor 110 may set a recommended sequencing area and display the recommended sequencing area on the pathological slide image 910 (or the heat map 921). Hereinafter, examples in which the processor 110 displays a sequencing area on the pathological slide image 910 or the heat map 921 will be described with reference to FIGS. 10A to 10D.

FIG. 10A is a diagram for describing an example in which a processor outputs a sequencing area, according to an embodiment.

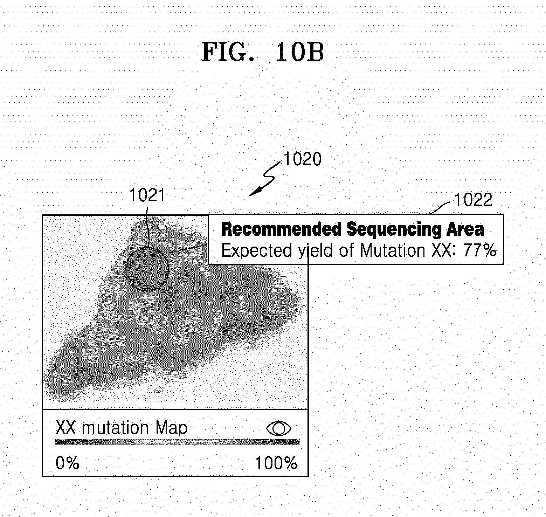

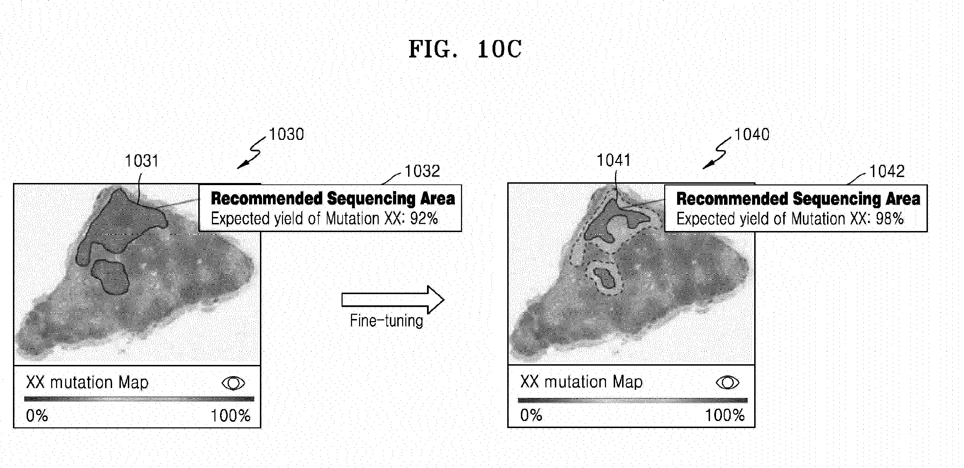

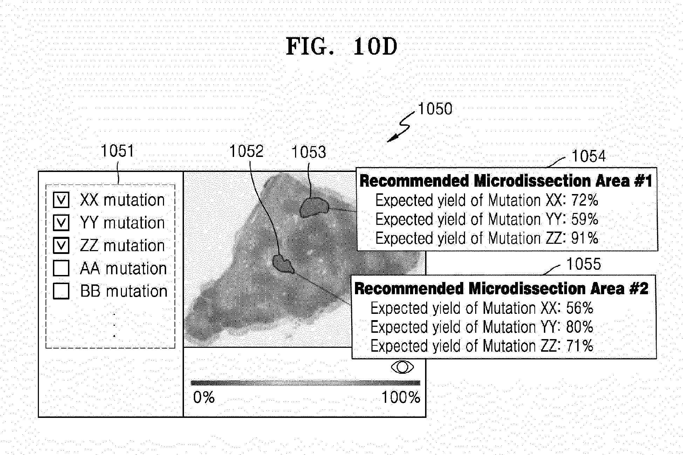

Referring to FIG. 10, in a screen 1010, a sequencing area 1011 may be displayed on a heat map. In addition, a yield 1012 that may be derived from the sequencing area 1011 may also be displayed on the screen 1010.

Here, the yield 1012 refers to an expected yield of mutation when separating the sequencing area 1011 from a subject. Accordingly, the user 30 may adjust the shape and size of the sequencing area 1011 such that the desired yield 1012 is derived.

FIG. 10B is a diagram for describing another example in which a processor outputs a sequencing area, according to an embodiment.

FIG. 10B illustrates a screen 1020 in which a sequencing area 1021 and a yield 1022 is indicated on a heat map.

There may be various methods of separating the sequencing area 1021 from a subject. For example, the methods of separating the sequencing area 1021 from the subject may include scalpel, punch, microdissection, and the like.

The processor 110 may set the sequencing area 1021 differently (i.e., adaptively) for each of the above-described methods, and output the yield 1022 for the sequencing area 1021. For example, for the scalpel method, the processor 110 may set the sequencing area 1021 considering the number of scalpel operations to separate the sequencing area 1021. As another example, for the punch method, the processor 110 may set the sequencing area 1021 considering the size of the punch. As another example, for the microdissection method, the processor 110 may set the sequencing area 1021 considering the type and size of a tool used for dissection.

FIG. 10C is a diagram for describing another example in which a processor outputs a sequencing area, according to an embodiment.

FIG. 10C illustrates an example of a method of outputting a sequencing area and allowing the user 30 to fine-tune the sequencing area, in order to be used in equipment capable of precisely dissecting the sequencing area, such as microdissection.

FIG. 10C illustrates an example in which sequencing areas 1031 and 1041 are modified and output according to manipulation of the user 30, and yields 1032 and 1042 are also changed and output accordingly.

For example, the sequencing area 1031 and the yield 1032 both set by the processor 110 may be displayed on a screen 1030. In this case, the user 30 may adjust the size, position, and direction of the sequencing area 1031, and the processor 110 may output the sequencing area 1041 modified by manipulation of the user 30, on a screen 1040. In addition, the changed yield 1042 according to the modified sequencing area 1041 may also be displayed on the screen 1040. Accordingly, the user 30 may fine-tune the sequencing areas 1031 and 1041 while comparing a critical yield for sequencing, with the yields 1032 and 1042.

FIG. 10D is a diagram for describing another example in which a processor outputs a sequencing area, according to an embodiment.

Referring to FIG. 10D, the processor 110 may output a screen 1050 where a plurality of mutations may be analyzed together.

For example, a list 1051 of a plurality of mutations may be displayed on the screen 1050, and check boxes, one or more of which may be selected by the user 30, may also be displayed. In addition, a plurality of sequencing areas 1052 and 1053 and their yields 1054 and 1055 may be displayed on the screen 1050.

When the user 30 selects one or more of the plurality of mutations, the processor 110 may predict and display the yields 1054 and 1055 of the selected mutations in each of the sequencing areas 1052 and 1053.

Although not illustrated in FIG. 10D, an optimal sequencing area for each mutation selected by the user 30 may be displayed on the screen 1050. For example, assuming that the user 30 selects an XX mutation and then switches to a YY mutation, an optimal sequencing area for the XX mutation may be displayed on the screen 1050, and then an optimal sequencing area for the YY mutation may be displayed. That is, showing and hiding a sequencing area may adaptively performed in response to a selection of the user 30.

In addition to the descriptions provided above with reference to FIGS. 10A to 10D, the processor 110 may additionally perform the following operations.

For example, the processor 110 may automatically detect an area in a heat map that satisfies a certain condition. For example, assuming that the user 30 wants to detect a mutation of gene A and the minimum probability of detecting a mutation of gene A is 60 %, the processor 110 may detect and output all areas in the heat map where the probability of detecting a mutation of gene A is 60 % or greater. In addition, the processor 110 may detect the area considering not only the minimum probability but also tumor purity.

As another example, the processor 110 may output a sequencing area set by the user 30, and a yield of the sequencing area. For example, the user 30 may directly set a sequencing area he or she wants, and the processor 110 may output the sequencing area set by the user, and a yield in the sequencing area.

As another example, the processor 110 may determine whether it is appropriate to perform sequencing on a subject corresponding to an entire heat map. For example, the processor 110 determines whether it is appropriate to perform sequencing on an entire area of the subject, by checking genotype scores for the entire area of the subject, or whether it is appropriate to perform sequencing on a partial area of the subject. For example, when tumor purity is low or the probability of a particular gene mutation is low, the processor 110 may determine a detailed sequencing area by referring to the heat map. Accordingly, the accuracy of NGS testing results may be improved.

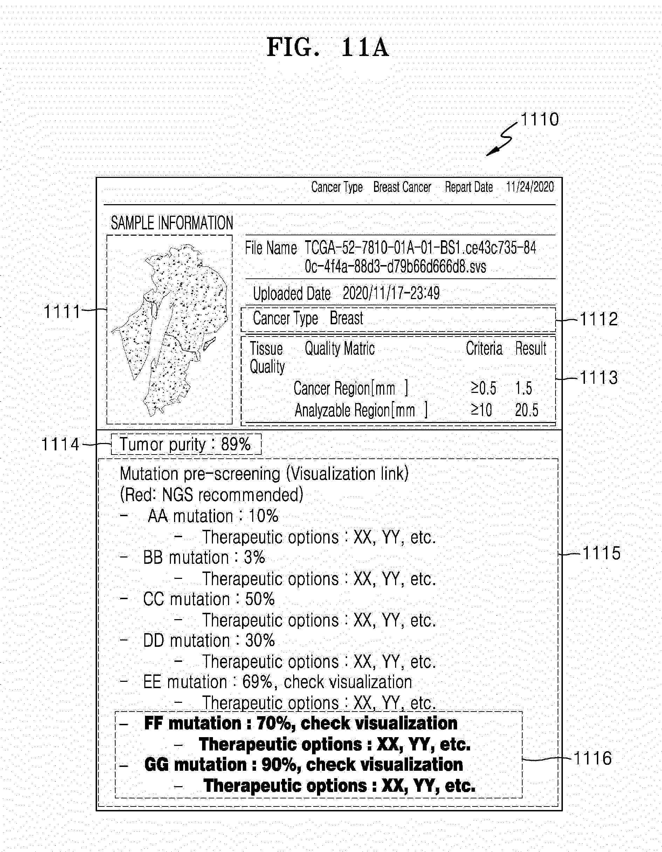

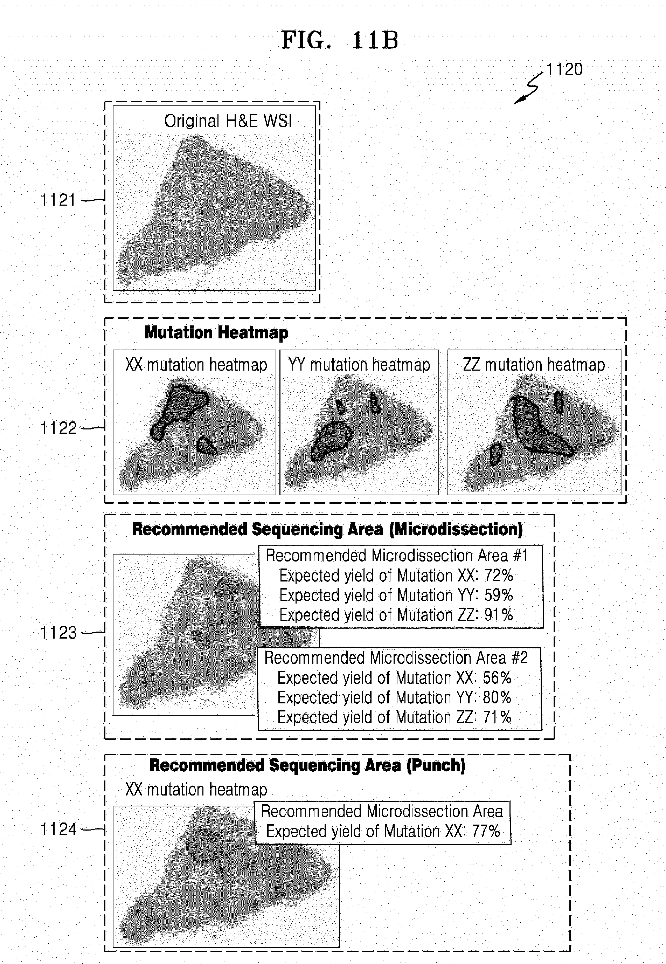

Meanwhile, the processor 110 may output a report including a sequencing area that is set based on medical information, and detailed information about the sequencing area. Hereinafter, examples of information included in a report will be described with reference to FIGS. 11A and 11B.

FIGS. 11A and 11B are diagrams illustrating examples of reports according to an embodiment.

Various pieces of medical information are described in reports 1110 and 1120 illustrated in FIGS. 11A and 11B. As described above with reference to FIG. 8, medical information and additional information based on the medical information may be collectively referred to as medical information. Thus, the information described in the reports 1110 and 1120 may include medical information and additional information.

For example, the reports 1110 and 1120 may be reports associated with NGS testing. In other words, the reports 1110 and 1120 may include not only various pieces of information about a subject, but also information about whether NGS testing is recommended, and information about a recommended sequencing area when performing NGS testing.

For example, the report 1110 may include a pathological slide image 1111 of a subject, a type 1112 of cancer that has developed in the subject, and information 1113 about the quality of the pathological slide image 1111. In addition, the report 1110 may include information about tumor purity 1114 of the subject, and information 1115 about probabilities of a plurality of gene mutations being discovered in the subject, and treatment methods. In particular, in the report 1110, information 1116 about gene mutations having a discovery probability greater than a threshold value and their treatment methods may be displayed to be distinguished from the information 1115. Accordingly, the user 30 may determine whether to perform NGS testing for the subject, through the report 1110.

In addition, the report 1120 may include various images 1121, 1122, 1123, and 1124. For example, the report 1120 may further include a pathological slide image 1121 of a subject, as well as various types of heat maps 1122, 1123, and 1124 described above with reference to FIGS. 9 to 10D. Through the heat maps 1122, 1123, and 1124, information about at least one genotype (e.g., a genotype score) may be confirmed, and at least one sequencing area and an expected yield of mutation corresponding thereto may also be confirmed. Accordingly, the user 30 may determine whether to perform NGS testing on the subject, through the report 1120.

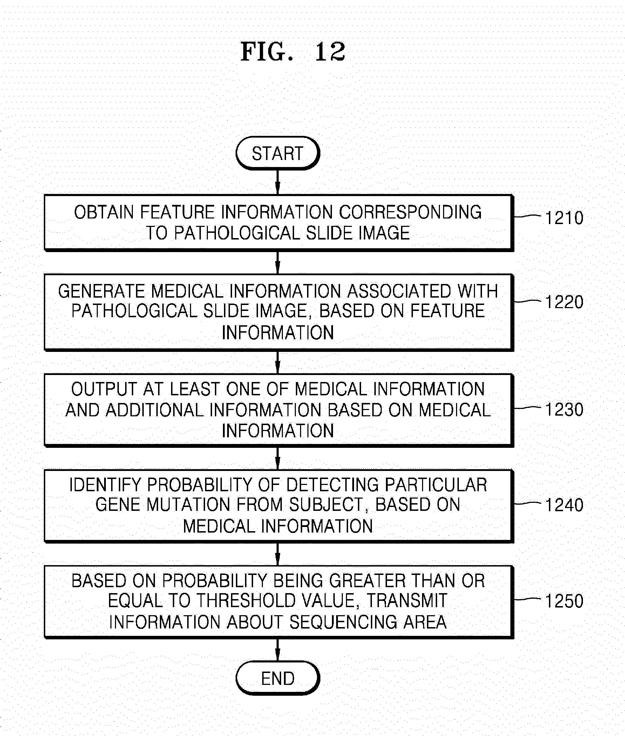

FIG. 12 is a flowchart for describing another example of a method of generating medical information from a pathological slide image, according to an embodiment.

Operations 1210 to 1230 of FIG. 12 are the same as operations 310 to 330 of FIG. 3. Thus, detailed descriptions of operations 1210 to 1230 will be omitted below.

In operation 1240, the processor 110 identifies a probability of detecting a particular gene mutation from a subject, based on medical information.

As described above with reference to FIGS. 10A to 10D, the processor 110 may identify a probability of detecting a particular gene mutation from the subject, based on genotype information (e.g., a genotype score).

In operation 1250, based on the probability identified through operation 1240 being greater than or equal to a threshold value, the processor 110 transmits information about the sequencing area.

For example, when it is determined that the probability of detecting the particular gene mutation is high, information about an area to be dissected from a slide (i.e., a sequencing area) may be transmitted to a device (e.g., a sequencing system) at an institution that performs NGS testing (e.g., a sequencing laboratory), and permission to view a result of analysis of the sequencing area by the processor 110 may be granted.

In addition, although not illustrated in FIG. 12, medical information and additional information generated by the processor 110 may be utilized as follows.

For example, they may be used for pre-screening of patients to be enrolled in a clinical trial. For example, the medical information and the additional information may be used to determine whether a patient is eligible to participate in a clinical trial as an experimental group for development of a new drug. As another example, they may be used for pre-screening before a genomic test for finding a treatment method suitable for a patient.

For example, the above-described pre-screening may be performed through the following process.

First, the test may be performed in a hospital or a laboratory where a pathological slide image of the patient is generated. When the pathological slide image is generated, analysis of the pathological slide image may be performed. For example, a result of the analysis may be generated in the form of i) tumor purity and a genotype score, ii) tumor purity and a heat map for each gene, or the like. Thereafter, one or more gene mutations may be selected based on probabilities of detecting the respective gene mutations. Thereafter, when it is determined that the probability of detecting a particular gene mutation is high, information about an area to be dissected from a slide (i.e., a sequencing area) may be transmitted to an institution that performs NGS testing, and permission to view a result of analysis of the sequencing area may be granted. When a slide arrives at the institution that performs NGS testing, the entire slide may be analyzed, or a selected region of interest of the slide may be analyzed.

As another example, the medical information and the additional information may be used to select a patient group in a clinical trial. For example, they may be used to receive conditions (e.g., a patient with an X mutation or a patient with a Y mutation but no Z mutation) from the user 30, and extract a list of patients who satisfy the conditions, in order to output a patient list.

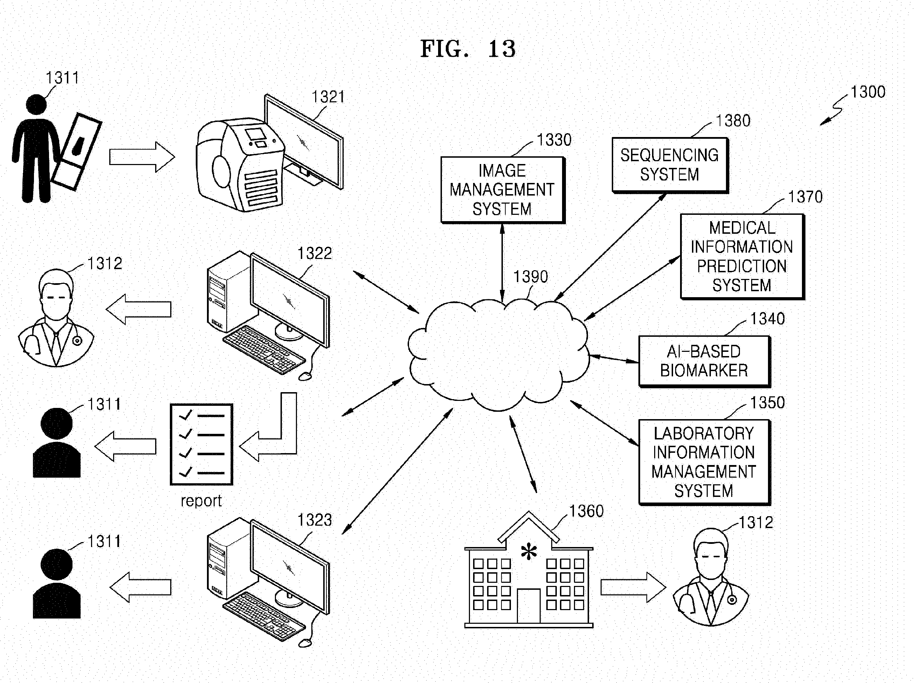

FIG. 13 is a diagram for describing another example of a system for generating medical information from a pathological slide image.

Referring to FIG. 13, a system 1300 is an example of a system and a network for preparing, processing, and reviewing slide images of tissue specimens by using a machine learning model.

According to various embodiments of the present disclosure, the methods described above with reference to FIGS. 2A to 12 may be performed by at least one of user terminals 1322 and 1323, an image management system 1330, an artificial intelligence (Al)-based biomarker analysis system 1340, a laboratory information management system 1350, a hospital or laboratory server 1360, a medical information prediction system 1370, and a sequencing system 1380, or a combination thereof.

A scanner 1321 may obtain a digitized image from a tissue sample slide generated by using a tissue sample of a subject 1311. For example, each of the scanner 1321, the user terminals 1322 and 1323, the image management system 1330, the Al-based biomarker analysis system 1340, the laboratory information management system 1350, the hospital or laboratory server 1360, the medical information prediction system 1370, and/or the sequencing system 1380 may be connected to a network 1390, such as the Internet, through one or more computers, servers, and/or mobile devices, or may communicate with a user 1312 through one or more computers, and/or mobile devices.

The user terminals 1322 and 1323, the image management system 1330, the Al-based biomarker analysis system 1340, the laboratory information management system 1350, the hospital or laboratory server 1360, the medical information prediction system 1370, and/or the sequencing system 1380 may generate, or otherwise obtain from another device, tissue samples, tissue sample slides, digitized images of tissue sample slides, or any combination thereof of one or more subjects 1311. In addition, the user terminals 1322 and 1323, the image management system 1330, the Al-based biomarker analysis system 1340, the laboratory information management system 1350, the hospital or laboratory server 1360, the medical information prediction system 1370, and/or the sequencing system 1380 may obtain any combination of subject-specific information, such as the age, medical history, a cancer treatment history, a family history, a past biopsy record, or disease information of the subject 1311.

The scanner 1321, the user terminals 1322 and 1323, the image management system 1330, the laboratory information management system 1350, and/or the hospital or laboratory server 1360 may transmit digitized slide images and/or subject-specific information to the medical information prediction system 1370 and/or the sequencing system 1380 through the network 1390. The medical information prediction system 1370 may include one or more storage devices (not shown) for storing received images and data. In addition, the medical information prediction system 1370 may include a machine learning model repository that stores a machine learning model trained to process the received images and data. For example, the medical information prediction system 1370 may include a machine learning model that is trained to predict, from a pathological slide image of the subject 1311, at least one of information about at least one cell, information about at least one area, information associated with a biomarker, medical diagnostic information, information about a genotype, information about a sequencing area of a tissue slide derived from the information about the genotype, detailed information associated with the sequencing area, and/or medical treatment information.

The scanner 1321, the user terminals 1322 and 1323, the medical information prediction system 1370, the laboratory information management system 1350, and/or the hospital or laboratory server 1360 may transmit, to the image management system 1330 through the network 1390, a digitized slide image, subject-specific information, and/or a result of analyzing the digitized slide image. The image management system 1330 may include a repository for storing received images and a repository for storing analysis results.

In addition, according to various embodiments of the present disclosure, a machine learning model that is trained to predict, from a slide image of the subject 1311, at least one of information about at least one cell, information about at least one area, information associated with a biomarker, medical diagnostic information, and/or medical treatment information, may be stored and operate in the user terminals 1322 and 1323, the image management system 1330, the medical information prediction system 1370, and the like.

According to various embodiments of the present disclosure, some or all of operations of the medical information prediction system 1370 may be performed by not only the medical information prediction system 1370, but also the user terminals 1322 and 1323, the image management system 1330, and the laboratory information management system 1350.

Meanwhile, the medical information prediction system 1370 may include a genotype predictor or may serve as the genotype predictor by itself.

Hereinafter, an example in which the medical information prediction system 1370 interacts with other components of the system 1300 will be described.

The medical information prediction system 1370 may receive an image of a tissue sample of the subject 1311 generated by the scanner 1321 (e.g., a H&E slide image or an IHC image), and an image of a tissue sample of the subject 1311 stored in the image management system 1330.

The medical information prediction system 1370 may derive information about a genotype (e.g., gene mutation information, heat map information for each mutation, or information about a particular gene expressed on a slide) by analyzing the image of the tissue sample of the subject 1311.

In detail, the medical information prediction system 1370 may include one or more machine learning models configured to receive an image of a tissue sample and predict information about a genotype. When receiving an image of a tissue sample, the medical information prediction system 1370 may check the analyzability of the image and, when the image is analyzable, extract features of the tissue slide, by using a machine learning model or a preset algorithm. The medical information prediction system 1370 may predict information about a genotype from the extracted features, by using a machine learning model.

When identifying the patient's high probability of a gene mutation (e.g., a high mutation score) based on the derived information about the genotype, the medical information prediction system 1370 may transmit the information about the genotype and/or an image of a tissue sample of the subject 1311, to the sequencing system 1380, in order to perform NGS testing.

The medical information prediction system 1370 may transmit the derived information about the genotype to the user terminals 1322 and 1323 such that the information is delivered to the user 1312. The transmitted information about the genotype may be used to write a report to be provided to the subject 1311. Meanwhile, the medical information prediction system 1370 may directly generate a report including the derived information about the genotype (e.g., the reports of FIGS. 11A and 11B), and transmit the report to the user terminals 1322 and 1323.

The medical information prediction system 1370 may transmit the derived information about the genotype to the hospital or laboratory server 1360 that studies tissue samples of the subject 1311.

The medical information prediction system 1370 may use the derived information about the genotype to extract a sequencing area, which is an area of the tissue slide for performing NGS testing. The sequencing area may be the entire tissue sample of the subject 1311 or a portion thereof. In addition, the medical information prediction system 1370 may derive detailed information about the extracted sequencing area.

When identifying the patient's high probability of a gene mutation, the medical information prediction system 1370 may transmit the extracted sequencing area and/or detailed information about the extracted sequencing area to the sequencing system 1380.

The information about the extracted sequencing area may be transmitted to the user terminals 1322 and 1323 and provided to the user 1312. The transmitted information about the sequencing area may be used to write a medical report to be provided to the subject 1311. Meanwhile, the medical information prediction system 1370 may directly generate a report including the derived sequencing area and/or detailed information about the sequencing area (e.g., the reports of FIGS. 11A and 11B), and transmit the report to the user terminals 1322 and 1323.

The user 1312 (e.g., a doctor/researcher, a hospital, a laboratory, or a patient) may arbitrarily adjust the size and shape of the sequencing area by using manipulable interfaces of the user terminals 1322 and 1323. The medical information prediction system 1370 may check the adjusted shape and size of the sequencing area to calculate/predict a yield in real time, and transmit the yield to the user terminals 1322 and 1323.

The information about the extracted sequencing area may be transmitted to the hospital or laboratory server 1360 that studies tissue samples of the subject 1311 and used for research, etc.

The medical information prediction system 1370 may upload/store images of tissue samples, derived information about genotypes, extracted sequencing areas, and/or detailed information about the sequencing areas, to the image management system 1330. The image management system 1330 may provide information to the user 1312 (e.g., a doctor/researcher, a hospital, a laboratory, or a patient), based on a query or a request of the user terminals 1322 and 1323 to manage the stored information.

As described above, the user terminal 10 may accurately and quickly generate medical information such as gene mutations or various biomarkers, and additional information based on the medical information, from a pathological slide image.

In addition, by combining information about an object extracted from the pathological slide image with feature vectors extracted from the pathological slide image, medical information may be generated by using various patterns and features in the pathological slide image. Thus, the medical information generated from the pathological slide image may be guaranteed to have high accuracy.

In addition, as the medical information includes information about various gene mutations (i.e., genotype information), targeted anti-cancer treatment may be performed quickly and accurately at a low cost. For example, the user terminal 10 may predict medical information (e.g., a KRAS G12C mutation) with high accuracy and stability by using a machine learning model and a pathological slide image. Thus, the medical information predicted by the user terminal 10 may be useful for NGS testing for targeted treatment of NSCLC or the like.

In addition, the user terminal 10 may generate a heat map or a distribution of various pieces of gene mutation information (i.e., genotype information), and generate a report including medical information and additional information. Thus, various pieces of information provided by the user terminal 10 may be useful for designing NGS testing or monitoring a patient in need of targeted anti-cancer treatment.

Meanwhile, the above-described method may be written as a computer-executable program, and may be implemented in a general-purpose digital computer that executes the program by using a computer-readable recording medium. In addition, the structure of the data used in the above-described method may be recorded in a computer-readable recording medium through various units. The computer-readable recording medium includes a storage medium, such as a magnetic storage medium (e.g., ROM, RAM, a universal serial bus (USB) drive, a floppy disk, a hard disk, etc.) and an optically readable medium (e.g., a CD-ROM, a DVD, etc.).

It will be understood by those of skill in the art that the present disclosure may be implemented in a modified form without departing from the intrinsic characteristics of the descriptions provided above. Therefore, the disclosed methods should be considered in an illustrative rather than a restrictive sense, and the scope of the present disclosure should be defined by claims rather than the foregoing description, and should be construed to include all differences within the scope equivalent thereto.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope as defined by the following claims.

## Claims

1. A computing device comprising:
at least one memory; and
at least one processor configured to
obtain feature information corresponding to a pathological slide image,
generate medical information associated with the pathological slide image based on the feature information, and
output at least one of the medical information and additional information based on the medical information.

2. The computing device of claim 1, wherein the at least one processor is further configured to obtain the feature information based on information about at least one object expressed in the pathological slide image.

3. The computing device of claim 2, wherein
the feature information comprises at least one first feature vector obtained by a feature embedding model, and
the feature embedding model is generated based on at least some layers of a first machine learning model configured to infer the information about the at least one object from the pathological slide image.

4. The computing device of claim 1, wherein the at least one processor is further configured to
generate a second feature vector by combining at least one first feature vector with information about at least one object expressed in the pathological slide image, and
generate the medical information based on the second feature vector.

5. The computing device of claim 4, wherein the at least one processor is further configured to perform pooling on at least one of the at least one first feature vector, the information about the at least one object, and a new feature vector that is a basis of the second feature vector.

6. The computing device of claim 4, wherein the at least one processor is further configured to generate a new feature vector by combining the second feature vector with a weight corresponding to the pathological slide image, and generate the medical information based on the new feature vector.

7. The computing device of claim 1, wherein the medical information comprises at least one of an immune phenotype, a genotype, a biomarker score, tumor purity, and a method of treating cancer expressed in the pathological slide image.

8. The computing device of claim 1, wherein the at least one processor is further configured to control a display device to display, on the pathological slide image, information indicating at least one genomic variation expressed in the pathological slide image.

9. The computing device of claim 1, wherein the at least one processor is further configured to control a display device to display, on the pathological slide image, a sequencing area that is set based on the medical information.

10. The computing device of claim 9, wherein the sequencing area is adaptively set for each of methods of separating the sequencing area from a subject.

11. The computing device of claim 9, wherein the at least one processor is further configured to control the display device to further output an expected yield of mutation when separating the sequencing area from a subject.

12. The computing device of claim 1, wherein the at least one processor is further configured to generate a report comprising a sequencing area that is set based on the medical information, and detailed information about the sequencing area.

13. The computing device of claim 1, wherein the at least one processor is further configured to identify, based on the medical information, a probability of detecting a particular genetic mutation from a subject, and based on the probability being greater than or equal to a threshold value, transmit information about a sequencing area.

14. A method comprising:
obtaining feature information corresponding to a pathological slide image;
generating medical information associated with the pathological slide image based on the feature information; and
outputting at least one of the medical information and additional information based on the medical information.

15. The method of claim 14, wherein the obtaining comprises obtaining the feature information based on information about at least one object expressed in the pathological slide image.
